# FASCICULE DE BREVET EUROPEEN

(11) **EP 4 263 801 B1**
(45) Date de publication et mention de la délivrance du brevet: **05.02.2025**
(21) Numéro de dépôt: 21854933.5
(22) Date de dépôt: 17.12.2021
(51) Int. Cl.: C12N 1/20

(54) **MILIEU DE CULTURE, PROCEDE DE FABRICATION ET PROCEDE DE CULTURE BACTERIENNE**
KULTURMEDIUM, HERSTELLUNGSVERFAHREN UND VERFAHREN ZUR KULTIVIERUNG VON BAKTERIEN
CULTURE MEDIUM, METHOD OF MANUFACTURE AND METHOD OF BACTERIAL CULTURE

(30) Priorité: 18.12.2020 FR 2013684
(43) Date de publication de la demande: 25.10.2023
(73) Titulaire: Université de Bourgogne, 21078 Dijon Cedex (FR); Institut National Superieur des Sciences Agronomiques de l'Alimentation et de l'Environnement, 21079 Dijon Cedex (FR); Institut national de recherche pour l'agriculture, l'alimentation et l'environnement, 75338 Paris Cedex 07 (FR); Institut National des Sciences et Industries du Vivant et de l'Environnement, 91120 Palaiseau (FR); Université Paris-Saclay, 91190 Gif-sur-Yvette (FR)
(72) Inventeur: BENEY, Laurent, 21000 Dijon (FR); RAISE, Audrey, 21000 Dijon (FR); DUPONT, Sébastien, 21490 Bretigny (FR); HUILLET, Eugénie, 92310 Sevres (FR)
(74) Mandataire: Novagraaf Technologies
(86) Numéro de dépôt international: PCT/FR2021/052397
(87) Numéro de publication internationale: WO 2022/129816

(56) Documents cités:
- WO-A1-2017/116235
- FU YOUSI ET AL: "Evaluation of the effects of four media on human intestinal microbiota culture in vitro", AMB EXPRESS, vol. 9, no. 1, 23 May 2019 (2019-05-23), XP055731511, DOI: 10.1186/s13568-019-0790-9

## Description

### Domaine technique de l'invention

La présente invention se rapporte à un milieu de culture bactérienne, à un procédé de fabrication du milieu de culture, ainsi qu'à un procédé de culture d'une bactérie, particulièrement efficace pour les cultures de bactéries sensibles et même très sensibles à l'oxygène ou anaérobies strictes. Le milieu de culture de l'invention permet par exemple de cultiver des bactéries anaérobies intestinales, par exemple de *Faecalibacterium prausnitzii* (*F. prausnitzii*)*.* Le milieu de culture de la présente invention permet notamment de fabriquer les nouvelles générations de probiotiques, notamment ceux destinés à prévenir et traiter les maladies inflammatoires chroniques de l'intestin dans le cadre strict des législations en matière d'alimentation humaine ou animale.

Dans le texte qui suit, le terme « Référence » suivi d'un chiffre renvoie à la liste des références bibliographies présentée après la partie « Exemples » ci-dessous.

### Art antérieur

A l'origine, les probiotiques (en anglais, « probiotics » ou « PBX ») sont des micro-organismes, bactéries ou levures, qui, ingérés en quantité adéquate et pouvant être intégrés à certains produits alimentaires pour les humains ou les animaux, sont censés conférer un bénéfice en matière de santé. Le concept de probiotique a ensuite été étendu à des traitements en vue de soigner ou prévenir des affections diverses. Il a en effet été démontré que les probiotiques ont de nombreux avantages pour la santé humaine et qu'ils jouent un rôle clé dans les processus digestifs normaux et dans le maintien de la santé humaine ou animale. Les applications agricoles des probiotiques, notamment la production animale, piscicole et végétale ont augmenté progressivement.

Toutefois, il existe un certain nombre d'incertitudes concernant les aspects technologiques, microbiologiques et réglementaires.

A titre d'exemple, on peut citer *F. prausnitzii,* la bactérie la plus abondante dans le microbiote intestinal. Elle représente de 3,5 à 5% des bactéries commensales. Cette bactérie est un des producteurs majeurs de butyrate, un acide gras à chaîne courte, qui participe au fonctionnement gastro-intestinal, et a de nombreux effets bénéfiques tels que la défense de la muqueuse intestinale, un effet moteur sur la motricité colique, un effet trophique sur l'épithélium intestinal et colique, un effet sur les défenses de la muqueuse intestinale par régulation de la sécrétion de mucus dans le côlon, permettant le maintien d'un pH constant au voisinage des cellules épithéliales, un effet sur les cellules néoplasiques colorectales, etc. (référence 1). Cependant, cette bactérie présente une extrême sensibilité à l'oxygène, ce qui rend délicates sa croissance et sa manipulation. La nécessité de la cultiver en chambre anaérobie rend techniquement difficile sa production et demande une grande expertise (référence 1). En outre, la législation en matière d'alimentation humaine est très stricte, ce qui limite encore la capacité actuelle de culture de ce microorganisme, et de manière plus générale des microorganismes présentant de telles sensibilités à l'oxygène. En effet, notamment, le milieu de culture doit être dépourvu de composés d'origine animale.

Le milieu de culture qui était le plus utilisé jusqu'à récemment est le milieu LYHBHI décrit dans le document Sokol, H. et al. (2008), F. prausnitzii is an anti-inflammatory commensal bacterium identified by gut microbiota analysis of Crohn disease patients, Proc. Natl. Acad. Sei. U. S. A.105, 16731-16736 (référence 2). Mais ce milieu de culture comprend des composés d'origine animale et ne permet donc pas de fabriquer des probiotiques conformes aux exigences en matière d'alimentation humaine.

Il existe donc un réel besoin d'un milieu de culture permettant à la fois de cultiver des bactéries sensibles, et même extrêmement sensibles à l'oxygène ou anaérobies strictes, et de produire des probiotiques conformes aux exigences strictes en matière d'alimentation humaine ou animale.

Le document WO 2017/116235 A1 (Caelus Pharmaceuticals B V (NL), 6 juillet 2017 (Référence 3) décrit des milieux de culture pour cultiver des micro-organismes intestinaux anaérobies obligatoires (notamment Eubacterium hallii), pour lesquels il est normalement difficile d'obtenir des rendements élevés en biomasse. Ces milieux ne comprennent pas de composants d'origine animale et permettent donc d'obtenir des préparations probiotiques.

Le document Fu Yousi et al : « Evaluation of the effects of four media on human instestinal microbiota culture in vitro", AMB Express, vol.9, n°1, 23 mai 2019 (Référence 4) décrit une étude de l'impact de quatre milieux sur des métabolites (acides gras à chaîne courte) et la composition du microbiote intestinal humain dans des cultures anaérobies en batch inoculées avec des échantillons de microbiote intestinal humain. -+-+

### Exposé de l'invention

La présente invention a précisément pour but de répondre à ces besoins en fournissant un milieu de culture bactérienne comprenant, par litre de milieu de culture, les composants suivants :
- de 20 à 40 g de peptones de pomme de terre,
- de 2 à 5 g d'acétate de métal alcalin anhydre,
- de 3 à 7 g d'un extrait de levure,
- de 2 à 16 g de maltose monohydraté, par exemple de 2 à 12 g de maltose monohydraté,
- de 0 à 1 g d'un sel soluble de la L-cystéine,
- de 0,006 à 0,015 g de biotine,
- de 0,0005 à 0,0015 g d'acide pantothénique ou un sel de celui-ci ; et
- de 0,0015 à 0,0025 g de pyridoxal ou d'un sel de celui-ci.

Selon l'invention, l'acétate de métal alcalin peut être choisi parmi un acétate de sodium ou de potassium, de préférence anhydre, par exemple de l'acétate de sodium ou de potassium anhydre.

Selon l'invention, l'extrait de levure peut être par exemple un extrait autolytique de levure, par exemple un extrait lyophilisé, par exemple résultant de l'autolyse de cellules de levures de *Saccharomyces cerevisiae,* mis en croissance sur des mélasses, par exemple de betterave, de soja ou de canne à sucre. Il peut s'agir par exemple d'un extrait de levure de numéro CAS 8013-01-2.

Selon l'invention, le maltose monohydrate est de préférence le D-(+)-maltose monohydraté de numéro CAS 6363-53-7. Le maltose est la source de sucre dans le milieu de culture. Alternativement ou en mélange, on peut également utiliser le cellobiose ou d'autres di-saccharides ou encore les monosaccharides les composant.

Selon l'invention, le sel soluble de la L-cystéine est de préférence la L-cystéine chlorhydrate monohydratée. Ce composant est un composant réducteur, qui permet de capturer l'oxygène moléculaire et de maintenir un faible potentiel rédox dans le milieu de culture.

Selon l'invention, l'acide pantothénique peut être par exemple le sel D-pantothénate de calcium, forme active de la vitamine B5.

Selon l'invention, le pyridoxal ou le sel de celui-ci peut être par exemple du chlorhydrate de pyridoxal ou du phosphate de pyridoxal. Ce composant peut être remplacé par la pyridoxamine et la pyridoxine.

Les inventeurs ont constaté que ce milieu de culture de l'invention, qu'ils ont défini au cours de leurs nombreuses expérimentations et travaux, avec le trio particulier de biotine, d'acide pantothénique ou un sel de celui-ci et de pyridoxal, est particulièrement efficace pour les cultures de bactéries sensibles et même très sensibles à l'oxygène ou anaérobies strictes, alors même qu'il ne comprend pas de composés d'origine animale. Le milieu de culture de la présente invention fournit d'excellentes performances de culture et croissance bactérienne, identiques, voire supérieures, aux milieux de culture de culture de l'art antérieur comprenant des composés d'origine animale.

En outre, le milieu de culture de la présente invention présente de nombreux autres avantages relevés par les inventeurs au cours des expérimentations, notamment : il ne mousse pas, et ne nécessite donc pas l'addition d'agents anti-mousse ; il ne précipite pas, contrairement à certains milieux de l'art antérieur ; et le nombre de composants nécessaires pour une culture bactérienne de qualité est réduit, avec seulement huit composants.

En outre, les expérimentations réalisées montrent que les performances bactériennes en termes de production de composés probiotiques sont préservées, voire améliorées. Par exemple, dans le cas de cultures réalisées avec *F. prausnitzii* sur le milieu de culture de la présente invention, les mesures montrent que la production de butyrate dans le milieu est similaire voire supérieure à celle produite dans les milieux comprenant des composés d'origine animale, par exemple en comparaison avec un milieu de culture cervelle, coeur.

Le milieu de culture de la présente invention permet donc avantageusement de fabriquer de nouvelles générations de probiotiques à partir de bactéries anaérobies, y compris extrêmement sensibles à l'oxygène ou anaérobies strictes, y compris *F. prausnitzii,* notamment, mais pas seulement, destinés à prévenir et traiter les maladies inflammatoires chroniques de l'intestin, dans le cadre strict des législations en matière d'alimentation humaine ou animale. Le milieu de culture de la présente invention peut être utilisé avantageusement pour la culture de bactéries anaérobies, micro-aérophiles ou anaérobies strictes, Gram négatif ou positif, par exemple à des fins d'étude de laboratoire, de recherche de traitements favorisant la croissance ou destinés à combattre ces bactéries, par exemple dans l'alimentation et/ou dans le domaine du médicament. On peut citer à titre d'exemple de bactéries qui peuvent avantageusement être cultivées sur le milieu de la présente invention les bactéries anaérobies intestinales, par exemple *F. prausnitzii, Blautia, par exemple Blautia hydrogenotrophica et Blautia obeum.*

La présente invention se rapporte également à un procédé de fabrication d'un milieu de culture selon l'invention comprenant :
(a) mélange des peptones de pomme de terre, de l'acétate de métal alcalin anhydre, de l'extrait de levure, et du maltose monohydraté dans un volume V1 de milieu aqueux, de préférence de l'eau distillée, obtenant ainsi un premier mélange ;
(b) ajustement du pH du premier mélange à une valeur de 7 à 8,
(c) chauffage du premier mélange au pH ajusté obtenu à l'étape (b) à une température de 110° à 130°C pendant une durée de 15 à 30 minutes, suivi d'un refroidissement à une température comprise entre 0° et 60°C ;
(d) mélange du sel soluble de la L-cystéine, de la biotine, de l'acide pantothénique ou d'un sel de celui-ci, et du chlorhydrate de pyridoxal dans un volume V2 de milieu aqueux, de préférence de l'eau distillée, obtenant ainsi un deuxième mélange ;
(e) stérilisation du deuxième mélange ; et
(f) mélange du premier mélange refroidi à l'étape (c) avec le deuxième mélange obtenu à l'étape (e) ;
les quantités des composants et les volumes V1 et V2 étant déterminées de manière à obtenir les concentrations définies ci-dessus pour le milieu de culture bactérienne de la présente invention.

Selon l'invention, l'étape (c), qui peut être une étape d'autoclavage, est avantageusement réalisée après mélange avec le maltose.

Selon l'invention, l'étape (c) de chauffage du premier mélange au pH ajusté obtenu à l'étape (b) à une température de 110 à 130°C pendant une durée de 15 à 30 minutes, suivi d'un refroidissement à une température qui peut être de 0° à 60°C, peut être réalisée par autoclavage, suivant toute technique et avec tout appareil connu de l'homme du métier pour stériliser un milieu de culture sans pour autant modifier chimiquement ses composants.

Selon l'invention, le maltose monohydraté peut être ajouté dans l'étape (a), c'est-à-dire avant l'étape (c), ou dans l'étape (d) ou (f), c'est-à-dire après l'étape (c), de préférence dans l'étape (a).

Selon l'invention, l'étape (e) peut être réalisée comme l'étape (c), c'est-à-dire par chauffage du deuxième mélange obtenu à l'étape (d) à une température de 110° à 130°C pendant une durée de 15 à 30 minutes, suivi d'un refroidissement à une température de 0° à 60°C, ou alors par filtration à 0,2 µm .

Selon l'invention, l'étape (f) de mélange des premier et second mélanges est de préférence réalisée en milieu stérile, par exemple sous un poste de sécurité microbiologique.

Selon l'invention, le procédé peut également comprendre une étape (g) de dégazage, par exemple sous N₂H₂CO₂, par exemple pendant 15 minutes à 6 heures, suivant le volume à dégazer, par exemple pendant 2 à 6 heures pour un bioréacteur industriel. Selon l'invention, le dégazage peut également être un dégazage passif, par exemple pendant une nuit à plusieurs jours, de préférence sous enceinte anaérobie.

La présente invention se rapporte également à un procédé de culture d'une bactérie comprenant un ensemencement d'un milieu de culture selon l'invention au moyen de la bactérie, et une culture de la bactérie dans le milieu de culture à une température permettant sa croissance.

L'homme du métier connait les températures optimales de croissance bactérienne ou sait les déterminer, notamment à partir de simples courbes de croissance en fonction de la température. Lorsqu'il s'agit de bactéries intestinales, la température de culture est généralement de 37°C.

Selon l'invention, la bactérie peut être l'une des bactéries précitées, par exemple une bactérie anaérobie intestinale, *F. prausnitzii* ou une bactérie sensible à l'oxygène ou une bactérie anaérobie stricte.

Selon l'invention, s'agissant de culture de microorganismes anaérobies, voire anaérobies strictes, la culture est bien entendu réalisée en condition anaérobie ou anaérobie stricte. Les méthodes de culture anaérobie sont connues de l'homme du métier et applicables ici. Les bactéries anaérobies strictes ne peuvent se cultiver qu'en l'absence d'oxygène. Cela nécessite donc de limiter le contact avec l'air ambiant. On peut incuber le milieu de culture une fois ensemencé dans un environnement exempt d'oxygène, par exemple dans une enceinte anaérobie, qui est une chambre de type chambre de Freiter, en utilisant des mélanges gazeux pour créer l'anaérobie, par exemple N2 et/ou CO₂, ou dans une jarre anaérobie ou une poche plastique qui utilisent des catalyseurs chimiques pour créer l'anaérobie, c'est-à-dire une atmosphère inférieure à 1% d'O₂, voire à 0% d'O₂.

D'autres avantages apparaîtront encore à la lumière des exemples qui suivent, donnés à titre illustratif et non limitatif, en référence aux figures annexées.

### Brève description des figures

[Fig. 1] représente une courbe de mesure de l'évolution de la densité optique à 600 nm d'un milieu de culture selon l'invention, en fonction de la durée en heures (h) de culture d'une bactérie anaérobie de la flore intestinale.
[Fig. 2] représente une courbe de mesure de l'évolution du pH d'un milieu de culture selon l'invention, en fonction de la durée en heures (h) de culture d'une bactérie anaérobie de la flore intestinale.
[Fig. 3] représente une courbe de mesure de l'évolution du nombre d'unité formant colonie (UFC) par mL d'un milieu de culture selon l'invention, en fonction de la durée en heures (h) de culture d'une bactérie anaérobie de la flore intestinale.
[Fig. 4] représente une courbe de mesure de l'évolution de la concentration en butyrate dans un milieu de culture selon l'invention, en fonction de la durée en heures (h) de culture d'une bactérie anaérobie de la flore intestinale.
[Fig. 5] représente une courbe de mesure de l'évolution de la densité optique (DO), mesurée à 600 nm, à partir de cultures de Blautia MB9 dans un milieu de culture selon l'invention, en fonction de la durée en heures (h) de culture.
[Fig. 6] représente la courbe de mesures de l'évolution de la densité optique à 600 nm d'un milieu de culture selon l'invention, en fonction de la durée en heures (h) de culture de *F. prausnitzii.*

### EXEMPLES

### Exemple 1 : Préparation de milieux de culture selon l'invention

La table 1 ci-dessous liste les composants utilisés dans ces exemples de mise en oeuvre de l'invention.

**[Table 1]**

| **Numéro du composant** | **Composant** | **Exemple de produit utilisé** | **Exemple de référence commerciale** |
|---|---|---|---|
| **1** | Peptones de pomme de terre | Hydrolysat de protéine de pomme de terre ultra clair | Potato peptone E210, Organotechnie (marque déposée) SAS |
| **2** | Acétate de métal alcalin anhydre | Acétate de sodium anhydre sous forme de poudre, numéro CAS 127-09-3 | référence S2889 (référence commerciale), Sigma-Aldrich |
| **3** | Extrait de levure | Extrait sous forme de poudre obtenu à partir de Saccharomyces cerevisiae Numéro CAS 8013-01-2 | référence Y1000 (référence commerciale), Sigma-Aldrich |
| **4** | Maltose monohydraté | D-(+)-Maltose monohydrate de pomme de terre, numéro CAS 6363-53-7 | référence M5885 (référence commerciale), Sigma-Aldrich |
| **5** | Sel soluble de la L-cystéine | L-cystéine hydrochloride monohydrate, numéro CAS 7048-04-6 | référence C7880 (référence commerciale), Sigma-Aldrich |
| **6** | Biotine | D-biotine sous forme de poudre lyophylisée, numéro CAS 58-85-5 | référence B4501 (référence commerciale), Sigma-Aldrich |
| **7** | Acide pantothénique ou un sel de celui-ci | Sel hémicalcique de l'acide D-pantothénique | référence P2250 (référence commerciale), Sigma |
| **8** | Pyridoxal | Chlorhydrate de pyridoxal | référence P9130 (référence commerciale), Sigma |

La table 2 ci-dessous liste les différentes compositions des composants listés dans la Table 1 utilisés dans ces exemples de mise en oeuvre de l'invention.

**[Table 2]**

| **Composant** | **1** | **2** | **3** | **4** | **5** | **6** | **7** | **8** |
|---|---|---|---|---|---|---|---|---|
| **Milieu 1** | 20 | 3 | 7 | 12 | 0 | 0,006 | 0,0005 | 0,0015 |
| **Milieu 2** | 20 | 5 | 5 | 8 | 0,5 | 0,006 | 0,0005 | 0,0015 |
| **Milieu 3** | 40 | 2 | 3 | 2 | 0,5 | 0,006 | 0,0005 | 0,0015 |
| **Milieu 4** | 35 | 2 | 3 | 2 | 1 | 0,015 | 0,0015 | 0,0025 |
| **Milieu 5** | 30 | 3 | 5 | 4 | 0 | 0,008 | 0,0008 | 0,0020 |

Le procédé pour fabriquer le milieu de culture a été le suivant :
Par exemple dans des tubes de Hungate : dissolution des composants 1 à 4 dans un volume d'eau distillée qui a été choisi en fonction du volume final souhaité. Une partie du volume d'eau distillée a été mise de côté, dans cet exemple 1%. Le volume d'eau distillé a été de 1 L, et le volume mis de côté de 100 mL. Les composants 1 à 4 ont été ajoutés, chacun en la quantité permettant d'obtenir les concentrations précitées lorsque le milieu de culture est finalisé. Plusieurs milieux ont été fabriqués, avec des variations de concentrations indiquées. Un premier mélange a donc été à chaque fois constitué avec les composants 1 à 4. Puis, le pH de ce premier mélange a été ajusté à 7,4 avec de la soude à 10 M.

Le premier mélange a été chauffé à une température de à 120°C pendant 20 minutes dans un autoclave afin de le stériliser. Il a ensuite été laissé se refroidir. Pendant ce temps, les composants 5 à 8 sont dissouts dans le reste d'eau distillée, ici 100 mL (chacun en la quantité permettant d'obtenir les concentrations précitées lorsque le milieu de culture est finalisé), afin d'obtenir le second mélange. Ce second mélange a ensuite été stérilisé par filtration à 0,2 µm sous un poste de sécurité microbiologique.

Le second mélange est ensuite ajouté au premier mélange et l'ensemble mélangés à homogénéité sous le poste de sécurité microbiologique, puis soumis à un dégazage au moyen d'un mélange H₂CO₂ dans N₂ (BIO-301, Air Liquide) pendant 4 heures.

Par exemple dans un bioréacteur : les composés 1 à 4 sont dissouts dans l'eau distillée puis ce milieu « basal » est mis dans le bioréacteur. Le bioréacteur est ensuite autoclavé directement avec le milieu et les sondes. Ensuite, la solution filtrée de vitamines et de cystéine (composés 5 à 8) est ajoutée dans le bioréacteur via une canule en conditions stériles grâce à l'utilisation d'une flamme. Puis ensuite le milieu est dégazé directement dans la cuve pendant 30 minutes.

Différents milieux ont ainsi été fabriqués, dont les milieux 1 à 5 ci-dessus définis.

### Exemple 2 : culture de F. prausnitzii et performances

Différents milieux fabriqués comme à l'exemple 1 ont été ensemencés avec 2% à 4% en volume de pré-culture de *Faecalibacterium prausnitzii.* Ces volumes correspondent à des ensemencements permettant d'obtenir des concentrations initiales de bactéries dans les cultures comprises entre 1E+06 et 1E+08 UFC/mL.

Dans ces exemples, *F. prausnitzii* utilisé est la souche DSM 17677 (Hauduroy et al. 1937, Duncan et al. 2002 emend. Fitzgerald et al. 2018), de la German Collection of Microorganisms and Cell Cultures GmbH (Allemagne).

Tous les milieux et toutes les solutions liquides entrant en contact avec la bactérie ont avant utilisation été dégazés activement en faisant buller le mélange gazeux N₂H₂CO₂ à l'intérieur pendant 4 h. Ce mélange gazeux a également été utilisé à chaque fois qu'il était nécessaire d'établir ou de maintenir l'anaérobiose dans un récipient hermétique ou un espace clos.

Une fois les milieux ensemencés, ils ont été mis en culture strictement anaérobie à 37°C dans une Etuve Memmert, dans les conditions suivantes : anaérobiose conservée grâce aux tubes de Hungate, 37 °C établi grâce à l'étuve, dans un bioréacteur à température régulée par bio-contrôleur, anaérobiose conservée grâce au passage de N₂H₂CO₂.

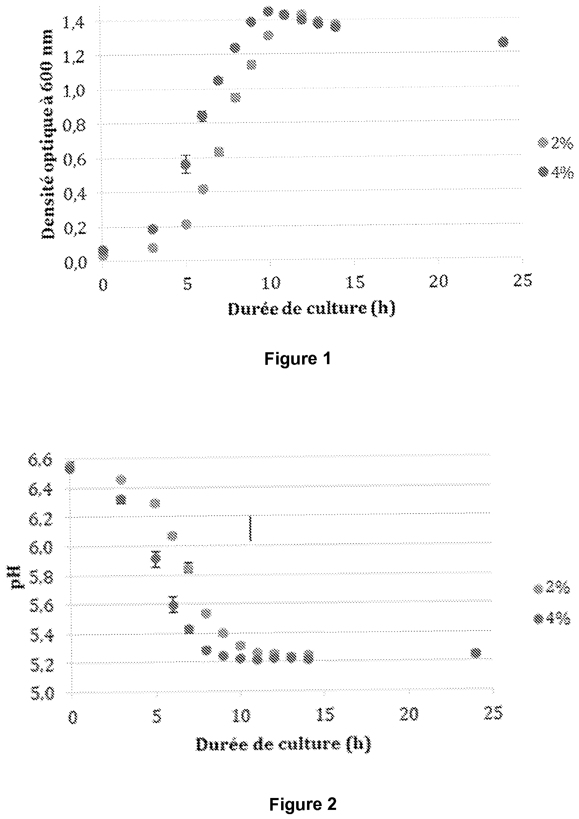

Les figures 1 à 4 annexées présentent différentes mesures de performance de culture sur les milieux de culture de la présente invention.

Les courbes représentées sur la figure 1 ont été obtenues à partir de cultures réalisées en tube de Hungate ensemencées à 2% ou 4% en volume. A chaque temps d'intérêt, 1 mL de culture était prélevé puis placé dans une cuve de spectrophotométrie de 2 mL. La densité optique de l'échantillon était ensuite mesurée à 600 nm avec un spectrophotomètre Helios Epsilon (marque de commerce - Thermoscientific). Chaque point correspond à une culture en tube de Hungate différente, utilisée uniquement pour obtenir la mesure au temps donné.

Sur cette figure, on constate que la croissance de *F. prausnitzii* est soutenue et entre en phase exponentielle plus rapidement lorsque les cultures sont ensemencées à 4%.

Les courbes représentées sur la figure 2 ont été obtenues à partir de cultures réalisées en tube de Hungate ensemencées à 2% ou 4% en volume. A chaque temps d'intérêt, le contenu d'une culture en tube de Hungate était versé dans un collecteur d'échantillon de 40 mL puis le pH de l'échantillon était mesuré grâce à un pH-mètre CyberScan 500 (marque de commerce, Eutech instruments -ThermoFisher Scientific, USA). La sonde utilisée était une sonde HI 1053 (référence commerciale - HANNA Instruments, France). Chaque point correspond à une culture en tube de Hungate différente, utilisée uniquement pour obtenir la mesure au temps donné.

Sur cette figure, on constate que le pH baisse rapidement lors de la phase exponentielle de culture et se stabilise autour d'une valeur de 5,3 en phase stationnaire, pour les deux séries de culture. La baisse de pH commence cependant plus rapidement pour la série de culture réalisée avec un taux d'ensemencement de 4%.

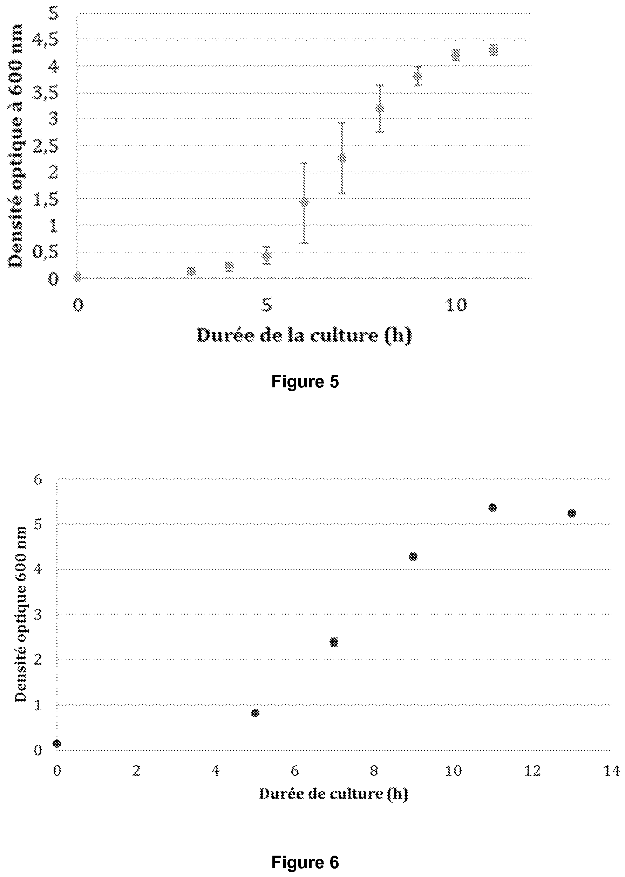

La figure 6 représente la courbe de mesures de l'évolution de la densité optique à 600 nm d'un milieu de culture selon l'invention, en fonction de la durée en heures (h) de culture de *F. prausnitzii.* Cette courbe a été obtenue dans les mêmes conditions opératoires que celles utilisées pour l'obtention de la courbe de la figure 1 avec un taux d'inoculation à 2%.

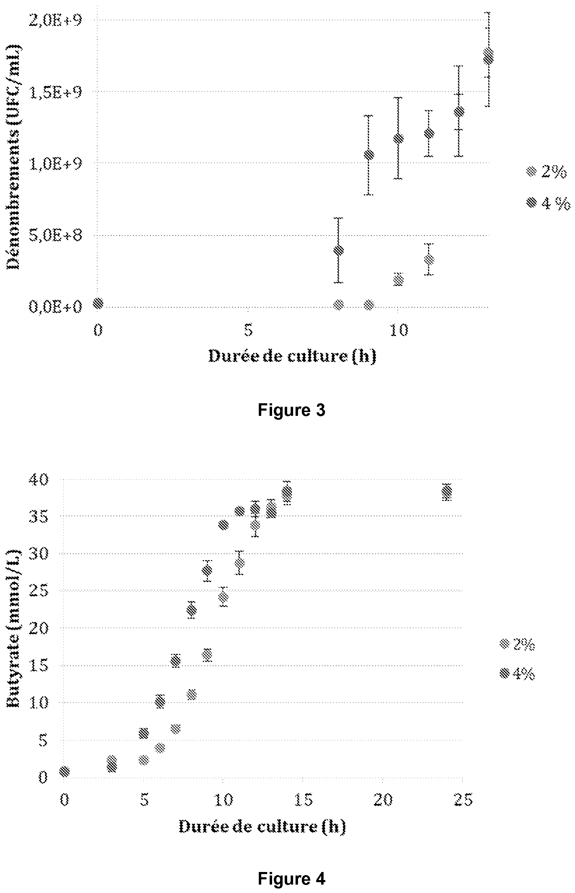

Les courbes représentées sur la figure 3 ont été obtenues à partir de cultures réalisées en tube de Hungate ensemencées à 2% ou 4% en volume. A chaque temps d'intérêt, les tubes de Hungate étaient ouverts sous une enceinte anaérobie Bactron 600-2 (marque commerciale - Sheldon manufacturing, USA) et la concentration en bactéries de la culture était évaluée par la méthode des Unité Formant Colonie (UFC/mL). Chaque point correspond à une culture en tube de Hungate différente, utilisée uniquement pour obtenir la mesure au temps donné.

Sur cette figure, on constate que les différents milieux conformes à la présente invention qui ont été testés ont permis d'obtenir environ 2×10⁹ UFC/mL lors de cultures en tubes de Hungate et en bioréacteur de 0,5 L utile. Cependant, la série de culture réalisée avec un taux d'ensemencement de 4% à une phase de latence plus courte et entre en phase exponentielle plus rapidement que la série de culture réalisée avec un taux d'ensemencement de 2%. Les valeurs maximales d'UFC/mL atteintes demeurent cependant identiques.

Les courbes représentées sur la figure 4 ont été obtenues à partir de cultures réalisées en tube de Hungate ensemencées à 2% ou 4% en volume. A chaque temps d'intérêt, 1 mL de suspension bactérienne a été prélevé dans un tube de Hungate puis 100 µL d'une solution à 5 % (v/v) d'acide orthophosphorique à 85 % (référence commerciale 20626.292, VWR) et 1 % (m/v) de chlorure mercurique (référence commerciale 4187302, Merck) lui ont été ajoutés. Les échantillons ont ensuite été homogénéisés et ont été conservés à -20 °C. Le jour de l'analyse, les échantillons ont été décongelés lentement puis 100 µL d'une solution d'étalon interne à 1 % (m/v) d'acide 4-méthyl valérique (référence commerciale 806088, Merck) ont été ajoutés à chaque échantillon. Les échantillons ont ensuite été mélangés puis centrifugés 10 min à 3800 g. Le surnageant de chaque échantillon a ensuite été récupéré à l'aide d'une seringue munie d'une aiguille puis filtré avec un filtre en cellulose régénérée à 0,2 µm (référence commerciale AF0-3203-12, Phenomenex (marque déposée)). Chaque filtrat a ensuite été déposé dans un flacon en verre Verex pour chromatographie en phase gazeuse (référence commerciale AR0-3700-13, Phenomenex (marque déposée)) et scellé avec un capuchon en polytétrafluoroéthylène (AR0-5710-13, Phenomenex (marque déposée)). Les échantillons ont été analysés par chromatographie en phase gazeuse couplée à un détecteur à ionisation de flamme (GC-FID) grâce à un chromatographe Clarus 500 (référence commerciale, Perkin Elmer, France), équipé d'un passeur et d'un injecteur automatiques. La séparation des acides gras a été réalisée sur une colonne capillaire Elite-FFAP (marque commerciale) (0,25 mm × 30 m × 0,25 µm, N9316352, Perkin Elmer, France). Pour l'analyse de chaque échantillon, 1 µL était prélevé et injecté en mode « splitless » (sans coefficient de partage) dans la colonne. La température d'injection était de 165 °C et le gaz vecteur utilisé était l'azote, à un débit de 1 mL/min. L'échantillon était ensuite chauffé pendant 13 min à 135 °C dans le four puis atteignait le détecteur à ionisation de flamme (FID), chauffé à 155 °C. Entre chaque injection, la seringue était rincée avec 1 µL d'eau distillée et 1 µL d'échantillon à analyser. Pour chaque échantillon, l'analyse a été réalisée en duplicata. Les chromatogrammes ont ensuite été analysés à l'aide du logiciel Azur (Référence commerciale - Perkin Elmer, France). Le butyrate a été identifié par comparaison de son temps de rétention à celui obtenu lors du passage d'une solution de standards (réalisée au laboratoire) analysée dans les mêmes conditions. Le temps de rétention se situait aux alentours de 6,31 secondes. Sur cette figure 4, on constate que *F. prausnitzii* a bien conservé sa capacité à produire du butyrate, ce qui démontre qu'il reste métaboliquement actif et qu'il conserve ses effets probiotiques dans un milieu de culture conforme à la présente invention. Les deux séries de culture permettent d'obtenir la même concentration maximale de butyrate produit, atteinte lors de l'entrée en phase stationnaire.

### Exemple 3 : culture de Blautia MB9

La courbe représentée sur la figure 5 illustre l'expérimentation réalisée dans cet exemple. Elle a été obtenue à partir de cultures de *Blautia* MB9 (*Blautia obeum*) dans milieu de culture et dans les conditions décrites dans l'exemple 2. A chaque temps d'intérêt, un volume de culture a été prélevé et la densité optique de l'échantillon était ensuite mesurée à 600 nm avec le spectrophotomètre Helios Epsilon (marque de commerce - Thermoscientific). Sur cette figure, on constate que la croissance de *Blautia* est soutenue. La phase exponentielle commence après 5h de culture. L'entrée en phase stationnaire se fait au bout de 10 h de culture. Des résultats similaires sont obtenus avec *Blautia hydrogenotrophica.*

### Références bibliographiques

**Référence 1** : Gastroentérologie clinique et biologique, Acides gras à chaîne courte : effets sur le fonctionnement gastro-intestinal et potentiel thérapeutique en Gastroentérologie ou « Short chain fatty acids (SCFA): effects on gastrointestinal function and therapeutic potential in Gastroenterology », vol. 23, N°6 et 7, Masson, Paris, Juillet 1999, p.761 et s.
**Référence 2 :** Sokol, H. et al. (2008), Faecalibacterium prausnitzii is an anti-inflammatory commensal bacterium identified by gut microbiota analysis of Crohn disease patients, Proc. Natl. Acad. Sei. U. S. A.105, 16731-16736.
**Référence 3 :** WO 2017/116235 A1 (CAELUS PHARMACEUTICALS B V (NL), 6 juillet 2017 (2017-07-06).
**Référence 4** : Fu Yousi et al : « Evaluation of the effects of four media on human instestinal microbiota culture in vitro", AMB Express, vol.9, n°1, 23 mai 2019.

## Revendications

1. Milieu de culture bactérien comprenant, par litre de milieu de culture, les composants suivants :
- de 20 à 40 g de peptones de pomme de terre,
- de 2 à 5 g d'acétate de métal alcalin anhydre,
- de 3 à 7 g d'un extrait de levure,
- de 2 à 16 g de maltose monohydraté,
- de 0 à 1 g d'un sel soluble de la L-cystéine,
- de 0,006 à 0,015 g de biotine,
- de 0,0005 à 0,0015 g d'acide pantothénique ou un sel de celui-ci ; et
- de 0,0015 à 0,0025 g de pyridoxal.

2. Milieu de culture selon la revendication 1, dans lequel l'acétate de métal alcalin est l'acétate de sodium anhydre.

3. Milieu de culture selon l'une quelconque des revendications précédentes, dans lequel l'extrait de levure est l'extrait de levure de numéro CAS 8013-01-2.

4. Milieu de culture selon l'une quelconque des revendications précédentes, dans lequel le maltose monohydrate est le D-(+)-maltose monohydraté de numéro CAS 6363-53-7.

5. Milieu de culture selon l'une quelconque des revendications précédentes, dans lequel le sel soluble de la L-cystéine est la L-cystéine chlorhydrate monohydratée.

6. Milieu de culture selon l'une quelconque des revendications précédentes, dans lequel l'acide pantothénique est le sel D-pantothénate de calcium.

7. Procédé de fabrication d'un milieu de culture selon l'une quelconque des revendications 1 à 6 comprenant :
(a) mélange des peptones de pomme de terre, de l'acétate de métal alcalin anhydre, de l'extrait de levure, et du maltose monohydraté dans un volume V1 de milieu aqueux, de préférence de l'eau distillée, obtenant ainsi un premier mélange ;
(b) ajustement du pH du premier mélange à une valeur de 7 à 8,
(c) chauffage du premier mélange au pH ajusté à l'étape (b) à une température de 110 à 130°C pendant une durée de 15 à 30 minutes, suivi d'un refroidissement à une température comprise entre 0 et 60°C ;
(d) mélange du sel soluble de la L-cystéine, de la biotine, de l'acide pantothénique ou d'un sel de celui-ci, et du chlorhydrate de pyridoxal dans un volume V2 de milieu aqueux, de préférence de l'eau distillée, obtenant ainsi un deuxième mélange ;
(e) stérilisation du deuxième mélange ; et
(f) mélange du premier mélange refroidi à l'étape (c) avec le deuxième mélange obtenu à l'étape (e) ;
les quantités des composants et les volumes V1 et V2 étant déterminées de manière à obtenir les concentrations définies dans la revendication 1.

8. Procédé de culture d'une bactérie comprenant un ensemencement d'un milieu de culture tel que défini dans l'une quelconque des revendications 1 à 6 au moyen de la bactérie ; et une culture de la bactérie dans le milieu de culture à une température permettant sa croissance.

9. Procédé de culture selon la revendication 8, la culture étant réalisée en condition anaérobie ou anaérobie stricte.

10. Procédé de culture selon la revendication 9, dans lequel la bactérie est une bactérie anaérobie intestinale, *Faecalibacterium prausnitzii* ou une bactérie sensible à l'oxygène ou une bactérie anaérobie stricte.

## Patentansprüche

1. Bakterielles Kulturmedium, das pro Liter Kulturmedium die folgenden Bestandteile enthält:
- 20 bis 40 g Kartoffelpeptone,
- 2 bis 5 g wasserfreies Alkalimetallacetat,
- 3 bis 7 g Hefeextrakt,
- 2 bis 16 g Maltosemonohydrat,
- 0 bis 1 g eines löslichen Salzes von L-Cystein,
- 0,006 bis 0,015 g Biotin,
- 0,0005 bis 0,0015 g Pantothensäure oder ein Salz davon; und
- 0,0015 bis 0,0025 g Pyridoxal.

2. Kulturmedium nach Anspruch 1, wobei das Alkalimetallacetat wasserfreies Natriumacetat ist.

3. Kulturmedium nach einem der vorhergehenden Ansprüche, wobei der Hefeextrakt ein Hefeextrakt mit der CAS-Nummer 8013-01-2 ist.

4. Kulturmedium nach einem der vorhergehenden Ansprüche, wobei das Maltosemonohydrat D-(+)-Maltosemonohydrat mit der CAS-Nummer 6363-53-7 ist.

5. Kulturmedium nach einem der vorhergehenden Ansprüche, wobei das lösliche Salz von L-Cystein L-Cysteinhydrochlorid-Monohydrat ist.

6. Kulturmedium nach einem der vorhergehenden Ansprüche, wobei die Pantothensäure das Calcium-D-Pantothenat-Salz ist.

7. Verfahren zur Herstellung eines Kulturmediums nach einem der Ansprüche 1 bis 6, umfassend:
(a) Mischen von Kartoffelpeptonen, wasserfreiem Alkalimetallacetat, Hefeextrakt und Maltosemonohydrat in einem Volumen V1 eines wässrigen Mediums, vorzugsweise destilliertem Wasser, um eine erste Mischung zu erhalten;
(b) den pH-Wert der ersten Mischung auf einen Wert von 7 bis 8 einstellen,
(c) Erhitzen der ersten pH-angepassten Mischung aus Schritt (b) auf 110 bis 130°C für 15 bis 30 Minuten, gefolgt von Kühlen auf 0 bis 60°C;
(d) Mischen von Maltosemonohydrat, dem löslichen Salz von L-Cystein, Biotin, Pantothensäure oder einem ihrer Salze und Pyridoxalhydrochlorid in einem Volumen V2 eines wässrigen Mediums, vorzugsweise destilliertes Wasser, wodurch eine zweite Mischung erhalten wird;
(e) Sterilisation der zweiten Mischung; und
(f) Mischen der in Schritt (c) gekühlten ersten Mischung mit der in Schritt (e) erhaltenen zweiten Mischung;
wobei die Mengen der Bestandteile und die Volumina V1 und V2 so bestimmt werden, dass man die in Anspruch 1 definierten Konzentrationen erhält.

8. Verfahren zur Kultivierung eines Bakteriums, umfassend das Beimpfen eines Kulturmediums nach einem der Ansprüche 1 bis 6 mit dem Bakterium und die Kultivierung des Bakteriums in dem Kulturmedium bei einer Temperatur, die sein Wachstum ermöglicht.

9. Kulturverfahren nach Anspruch 8, wobei die Kultur unter anaeroben oder streng anaeroben Bedingungen durchgeführt wird.

10. Kulturverfahren nach Anspruch 9, wobei das Bakterium ein anaerobes Darmbakterium, *Faecalibacterium prausnitzii* oder ein sauerstoffempfindliches Bakterium oder ein streng anaerobes Bakterium ist.

## Claims

1. Bacterial culture medium comprising, per liter of culture medium, the following components:
- 20 to 40 g potato peptones,
- 2 to 5 g of anhydrous alkali metal acetate,
- 3 to 7 g of yeast extract,
- 2 to 16 g maltose monohydrate,
- 0 to 1 g of a soluble salt of L-cysteine,
- 0.006 to 0.015 g biotin,
- from 0.0005 to 0.0015 g pantothenic acid or a salt thereof; and
- 0.0015 to 0.0025 g pyridoxal.

2. Culture medium of claim 1, wherein the alkali metal acetate is anhydrous sodium acetate.

3. Culture medium according to any one of the preceding claims, wherein the yeast extract is yeast extract of CAS number 8013-01-2.

4. Culture medium according to any one of the preceding claims, wherein the maltose monohydrate is D-(+)-maltose monohydrate with CAS number 6363-53-7.

5. Culture medium according to any one of the preceding claims, wherein the soluble salt of L-cysteine is L-cysteine hydrochloride monohydrate.

6. Culture medium according to any one of the preceding claims, wherein the pantothenic acid is the calcium D-pantothenate salt.

7. A method of manufacturing a culture medium according to any one of claims 1 to 6 comprising:
(a) mixing potato peptones, anhydrous alkali metal acetate, yeast extract and maltose monohydrate in a volume V1 of aqueous medium, preferably distilled water, thus obtaining a first mixture;
(b) adjust the pH of the first mixture to a value of 7 to 8,
(c) heating the first pH-adjusted mixture from step (b) to 110 to 130°C for 15 to 30 minutes, followed by cooling to 0 to 60°C;
(d) mixing maltose monohydrate, the soluble salt of L-cysteine, biotin, pantothenic acid or a salt thereof, and pyridoxal hydrochloride in a volume V2 of aqueous medium, preferably distilled water, thus obtaining a second mixture;
(e) sterilization of the second mixture; and
(f) mixing the first mixture cooled in step (c) with the second mixture obtained in step (e);
the quantities of the components and the volumes V1 and V2 being determined so as to obtain the concentrations defined in claim 1.

8. A method of culturing a bacterium comprising inoculating a culture medium according to any one of claims 1 to 6 with the bacterium; and culturing the bacterium in the culture medium at a temperature permitting its growth.

9. Culture process according to claim 8, the culture being carried out under anaerobic or strict anaerobic conditions.

10. The culture method of claim 9, wherein the bacterium is an anaerobic intestinal bacterium, *Faecalibacterium prausnitzii* or an oxygen-sensitive bacterium or a strict anaerobic bacterium.
